# EUROPÄISCHE PATENTANMELDUNG

(11) **EP 1 561 747 A2**
(43) Veröffentlichungstag der Anmeldung: **10.08.2005**
(21) Anmeldenummer: 04027837.6
(22) Anmeldetag: 24.11.2004
(51) Int. Cl.: C07C 313/04

(54) **Verfahren zur Herstellung von fremdsalzarmen Sulfinaten**

(30) Priorität: 24.11.2003 DE 10355088
(71) Anmelder: BASF AKTIENGESELLSCHAFT, 67056 Ludwigshafen (DE)
(72) Erfinder: Böhnke, Harald Dr., 67346 Speyer (DE); Frank, Jürgen Dr., 67065 Ludwigshafen (DE); Pütter, Hermann Dr., 67433 Neustadt (DE); Schelling, Heiner, 67281 Kirchheim (DE); Schneider, Reinhardt Dr., 67136 Fussgönheim (DE); Thate, Sven Dr., 67271 Neuleiningen (DE)
(74) Vertreter: Huhn, Michael, Dr.

(57) **Zusammenfassung**

Die vorliegende Erfindung bezieht sich auf ein Verfahren zur Herstellung von fremdsalzarmen Sulfinaten durch Reduktion von Dithionit-Salzen der Alkali- oder Erdalkalimetalle oder Ammonium mit Carbonylverbindungen oder Iminen, wobei das Verfahren bei einem pH-Wert von > 6 durchgeführt wird. Das molare Verhältnis Dithionitsalz zu Carbonyl- oder Iminverbindung beträgt dabei vorzugsweise 0,75 bis 1,3.

Das Verfahren gestattet die Herstellung fremdsalzarmer Sulfinat-Produkte. Gewünschtenfalls können entstandene Fremdsalze mit üblichen Methoden weiter abgetrennt werden. Das Verfahren eignet sich insbesondere zur Herstellung von Hydroxymethylsulfinat.

## Beschreibung

Die vorliegende Erfindung bezieht sich auf ein Verfahren zur Herstellung von fremdsalzarmen Sulfinaten durch Reduktion von Dithionit-Salzen der Alkali- oder Erdalkalimetalle oder Ammonium mit Carbonyl- oder Iminverbindungen bei pH-Werten > 6. Durch Einhalten von bestimmten Konzentrationsverhältnissen der Edukte zueinander werden fremdsalzarme Produkte erzeugt, wobei die Fremdsalze durch übliche Verfahren leicht weiter abgetrennt werden können.

Sulfinate haben als Reduktionsmittel und als Radikalerzeuger vielseitige Verwendung in der Technik gefunden. Beispiele dafür sind Färbe- und Entfärbeprozesse sowie Polymerisationen.

Sulfinate werden generell durch Umsetzung von Dithionit-Salzen mit Aldehyden in Gegenwart von Alkali hergestellt. Sämtliche beschriebenen Herstellungsprozesse weisen jedoch den Nachteil auf, dass die erhaltenen Sulfinat-Lösungen Stoffe enthalten, die während des Herstellungsprozesses gebildet werden. Häufig werden diese Lösungen ohne weitere Abtrennung bzw. Aufarbeitung verkauft, wobei jedoch eine relativ geringe massenbezogene Wirkungskraft des Produkts resultiert. Eine Abtrennung der Nebenprodukte ist generell möglich, jedoch im Allgemeinen sehr aufwendig. Durch den Salzanfall entstehen hohe Herstellungs- bzw. Produkttransportkosten.

Beispielsweise entstehen bei der Herstellung von Sulfinaten nach dem Zinkstaubverfahren (Reduktion von SO₂ mit Zn über den Zwischenstoff Zn-Dithionit und anschließende Umsetzung mit Aldehyden) Zinkoxide bzw. Zinkhydroxide als Koppelprodukte. Diese müssen aufwendig aus der Produktlösung abgetrennt werden, wobei die oftmals geringe Marktnachfrage des Koppelproduktes die Wirtschaftlichkeit des gesamten Herstellungsprozesses beeinflusst. Weiterhin enthält die erhaltene Produktlösung bei diesem Verfahren stets Zn-Ionen als Verunreinigungen, die bei Anwendung eines solchen Sulfinat-Produkts häufig wegen nachteiliger Effekte unerwünscht sind.

Außer durch Umsetzung mit Zn kann SO₂ elektrolytisch in Dithionit überführt werden. Dabei liegen keine Zn-Ionen in dem erhaltenen Produktgemisch bzw. dem isolierten Dithionit vor. Zn-Salze sind generell schwierig abzutrennen durch Filtration, was bei der Isolierung von aus Dithioniten erhaltenen Sulfinaten deren Isolierung erschwert.

Sulfinate können direkt durch Umsetzung von Natriumdithionit mit Aldehyden und Basen hergestellt werden. Die dabei entstehenden hauptsächlichen Nebenprodukte sind Sulfite und Sulfonate, die ebenfalls die massenbezogene Wirkungskraft des Produkts verringern bzw. abgetrennt werden müssen. Wirtschaftliche Verfahren zur Anreicherung des Sulfinates und Abtrennung der Fremdsalze sind bisher in der Literatur nicht beschrieben.

Die Herstellung von Sulfinaten aus Natriumdithionit und NaOH durch langsame Zugabe von Aldehyden ist beschrieben, siehe Mulliez, Tetrahedron 49, 12, pp. 2469-2476 (1993). Über die Zusammensetzung der erhaltenen Produktgemische wird hinsichtlich des Verhältnisses von Sulfinat/Sulfit zu Sulfonat keinerlei Aussage getroffen. Nicht beschrieben wird die Herstellung von Hydroxymethylsulfinat auf die genannte Weise. Weiterhin wird mit kommerziell erhältlichem Dithionit, das in hoher Reinheit vorliegt, gearbeitet.

Es sind bis jetzt keine wirtschaftlichen Verfahren bekannt, die eine effektive Anreicherung des Sulfinats von Produktgemischen ermöglichen, wie sie nach den bisherigen, im Stand der Technik bekannten Verfahren erhalten wurden. Dabei liegt ein hoher Anteil an Verunreinigungen bereits im Dithionit vor, das nach den vorstehend beschriebenen Verfahren hergestellt wird. Diese Verunreinigungen werden weitergeschleppt beziehungsweise können weiterreagieren. Zwar ist es bekannt, bei bestimmten Sulfinaten diese durch Zugabe polarer, wasserlöslicher organischer Lösungsmittel in dem erhaltenen Produktgemisch anzureichern, siehe US 6,211,400. Der Sulfinat-Gehalt im Produkt liegt aber nach Anreicherung immer noch niedrig, generell bei Werten von unter 80 %. Der bei der Aufreinigung eintretende Sulfinat-Verlust wird nicht näher ausgeführt.

Es wird weiterhin nach Verfahren gesucht, die Sulfinate ausgehend von Schwefeldioxid bzw. Dithioniten bereitstellen sollen. Dies soll ohne den Anfall größerer Mengen von Fremdsalzen gelingen. Vorzugsweise soll der unvermeidliche Anfall von Fremdsalzen so gering sein, dass eine Aufreinigung der erhaltenen Sulfinat-Produkte und ein Abtrennen der Fremdsalze bis zu einem an höchste Marktanforderung heranreichenden Grad von Reinheit mit üblichen Methoden möglich ist. Insbesondere soll dabei der Anfall von Sulfonaten gering sein.

Diese Aufgabe wird gelöst durch ein Verfahren zur Herstellung von fremdsalzarmen Sulfinaten durch Reduktion von Dithionit-Salzen der Alkalimetalle oder Erdalkalimetalle oder Ammonium mit Carbonylverbindungen oder Iminen bei einem pH-Wert > 6.

Durch die Reaktionsführung gemäß der vorliegenden Erfindung erfolgt die Sulfinat-Bildung mit hohen Ausbeuten und Selektivitäten, die unerwünschte Bildung der Nebenprodukte wird stark zurückgedrängt. Das erhaltene Sulfinat-Produkt weist also neben dem Wertprodukt-Sulfinat selbst nur eine geringe Menge an Fremdprodukten, insbesondere Fremdsalzen, auf.

Es wird eine hohe Relation Wertprodukt/Fremdsalz erreicht. Die erhaltenen Sulfinat-Produkte weisen eine hohe massenbezogene Wirkungskraft auf.

Unter dem Begriff "Fremdsalze" werden im Zusammenhang mit der vorliegenden Erfindung unerwünschte, neben dem Sulfinat-Hauptprodukt anfallende Salze verstanden. Zum einen sind dies Sulfite, die bei der Umsetzung von Dithionit mit Aldehyden nach der vorliegenden Erfindung zwangsläufig (Reaktionsstöchiometrie) entstehen. Der unerwartete Vorteil des erfindungsgemäßen Verfahrens liegt dabei darin, dass Sulfit in stöchiometrischer Menge entsteht und dies leicht abgetrennt werden kann. Zum anderen umfasst der Begriff "Fremdsalze" auch Salze, die durch Nebenreaktionen entstehen oder in dem Edukt Dithionit vorliegen (generell aus dessen Herstellungsprozess).

Das molare Verhältnis der Dithionit-Salze zu Carbonyl- oder Iminverbindung liegt bei Werten von 0,75 bis 1,3, vorzugsweise 0,95 bis 1,05, insbesondere 0,99 bis 1,02. Die genaue Dosierung der Edukte zueinander bringt vorteilhafte Resultate.

Der pH-Wert der Lösung liegt bei Werten von > 6, vorzugsweise 8 bis 13, insbesondere 9 bis 11.

Generell wird der pH-Wert auf die erfindungsgemäß erforderlichen Werte durch Zugabe von Alkali- oder Erdalkalimetallhydroxiden oder Stickstoffbasen eingestellt. Beispiele für die genannten Hydroxide umfassen NaOH, KOH, Ca(OH)₂, Mg(OH)₂, Ba(OH)₂. Als Stickstoffbasen eignen sich unsubstituierte und alkyl-, alkylaryl-, aryl- und arylalkylsubstituierte Ammoniumsalze, beispielsweise Tetramethylammonium- oder Tetraethylammoniumsalze. Weitere geeignete Stickstoffbasen sind Stickstoffheterocylcen, beispielsweise Imidazoliniumsalze oder Pyridiniumsalze. Bevorzugt ist die Verwendung von NaOH.

Das Dithionit wird generell in Form des Natrium-Salzes eingesetzt werden. Alternativ können andere Alkali- oder Erdalkalimetallsalze oder auch das Ammoniumsalz des Dithionits eingesetzt werden.

Das Dithionit kann auch als Zn-Salz vorliegen (erhalten nach den Reduktionsverfahren aus SO₂ und Zn). Vorzugsweise ist das Dithionit frei von Zn-Salzen.

Im Verfahren nach der vorliegenden Erfindung kann ein Dithionit in Form des isolierten entsprechenden Salzes eingesetzt werden, wobei das Salz gereinigt sein oder mit Verunreinigungen vorliegen kann. Alternativ kann als erfindungsgemäße Verfahren als integrierter Prozess durchgeführt werden, bei dem zunächst SO₂ bzw. Sulfit-Salze in Dithionit überführt werden und dies direkt im Anschluss, ohne Isolierung, erfindungsgemäß zu Sulfinaten umgesetzt wird.

Dabei kann die Reduktion des SO₂ bzw. Sulfits beispielsweise durch Zn oder elektrolytisch erfolgen. Im Rahmen der vorliegenden Erfindung ist dabei die elektrolytische Reduktion bevorzugt.

Es wurde gefunden, dass durch Elektrolyse erhaltene Dithionit-Lösungen problemlos in dem erfindungsgemäßen Verfahren eingesetzt werden können, da durch die Reaktionsführung kein weiteres unerwünschtes Fremdsalz entsteht und die bei der Herstellung des Dithionits anfallenden Salze (größtenteils Sulfit) mit den Fremdsalzen des erfindungsgemäßen Verfahrens problemlos abgetrennt werden können. Es erübrigt sich somit eine Aufreinigung des Dithionits.

Das erhaltene Sulfinat kann in Form des Alkali-, Erdalkali- oder Ammoniumsalzes erhalten werden, vorzugsweise als Na-Salz.

Als Carbonylverbindung eignen sich prinzipiell sämtliche Aldehyde und Ketone, sowohl aliphatische und aromatische als auch ein- und mehrwertige. Beispiele umfassen Formaldehyd, Acetaldehyd, Aceton, Hydroxyaceton, Cyclohexanon, Adipoin, Glyoxal und Glyoxylsäure. Bevorzugt ist Formaldehyd.

Imine entstehen aus der Reaktion von Aminen, die ein abstrahierbares H-Atom enthalten, mit Aldehyden und Keton. Generell sind sämtliche Imine für den Einsatz im erfindungsgemäßen Verfahren geeignet. Bevorzugt sind von NH₃ abgeleitete Imine. Insbesondere bevorzugt sind die Imine hergestellt aus Formaldehyd und NH₃, Methylamin, Ethylamin, Ethanolamin oder Ethylendiamin sowie die von der BASF unter dem Namen Cyclanon® Eco vertriebenen Imine.

Das erfindungsgemäße Verfahren eignet sich insbesondere zur Herstellung von Hydroxymethylsulfinat bzw. dessen Salzen. Dabei ist es weiterhin bevorzugt, wenn das eingesetzte Dithionit durch elektrolytische Reduktion von SO₂ bzw. Sulfiten hergestellt wurde.

Die Trennung erfolgt mit dem Fachmann bekannten Methoden, beispielsweise Selektivfällung, Abtrennung im elektrischen Gradienten, vorzugsweise Elektrodialyse oder Elektophorese, oder Abtrennung durch Druckgradienten, beispielsweise Osmose, Nanofiltration, Umkehrosmose oder selektive Ionentauscher.

Die Selektivfällung erfolgt im Allgemeinen durch Zugabe von polaren, wasserlöslichen organischen Lösungsmitteln. Beispiele umfassen Ketone, Alkohole, Aldehyde, Amide, Sulfoxide, wasserlösliche cyclische Ether und wasserlösliche N-Heterocyclen.

Beispiele für geeignete Alkohole umfassen C₁-C₄-Alkohole, Diole, Glycerin. Beispiele für geeignete cyclische Ether umfassen Tetrahydrofuran THF und Dioxan. Ein Beispiel für einen geeigneten N-Heterocylcus ist N-Methylpyrrolidon NMP. Weitere Beispiele für geeignete Lösungsmittel sind Dimethylsulfoxid DMSO und Dimethylformamid DMF.

Bevorzugt ist die Zugabe von Alkoholen. Die meist bevorzugten Lösungsmittel, die zur Fällung eingesetzt werden, sind Methanol, Ethanol und Isopropanol.

Die ausgefällten Fremdsalze werden mit den üblichen, dem Fachmann bekannten Filtrationsmethoden abgetrennt. Die Filtration kann unter Normaldruck oder erhöhtem Druck erfolgen.

Eine weitere Möglichkeit zur Abtrennung der Fremdsalze liegt in der Elektrodialyse. Elektrodialytische Verfahren sind dem Fachmann bekannt. Sie ermöglichen eine Abtrennung unterschiedlich geladener Salze voneinander, generell durch Verwendung von ladungsspezifischen Membranen. Elektrodialytische Verfahren lassen sich bei dem erfindungsgemäßen Verfahren insbesondere dann anwenden, wenn das Substrat und das Fremdsalz unterschiedliche Ladungen aufweisen, beispielsweise wie im Fall von Sulfit/Hydroxymethylsulfinat. Das erfindungsgemäße Verfahren kann dabei so durchgeführt werden, dass Produkt und Fremdsalz unterschiedliche Ladungen aufweisen. Beispielsweise wird die Reaktion so durchgeführt, dass bei der Synthese von Hydroxymethylsulfinat das entstehende Fremdsalz hauptsächlich Sulfit ist.

Eine weitere Möglichkeit des Entfernens der Fremdsalze liegt in der Abtrennung durch Druckgradienten, beispielsweise Osmose, Umkehrosmose, Nanofiltration oder selektive Ionentauscher.

Aufgrund der hohen Selektivität des erfindungsgemäßen Verfahrens ist es möglich, das entstandene Fremdsalz, das frei von Schwermetallen und organischen Verunreinigungen ist, nach Abtrennung weiter zu verwenden. Sie lassen sich gegebenenfalls als solche als Wertprodukt vermarkten, oder können zur Herstellung anderer Produkte verwendet werden, beispielsweise zur Herstellung von Dithionit, im Falle von Sulfitsalzen. Beispielsweise können Sulfitsalze elektrochemisch zu Dithionit-Lösungen umgesetzt werden, etwa im Amalgam- oder Membranverfahren.

Die Erfindung wird nun in den nachfolgenden Beispielen erläutert.

### Beispiele

### Beispiel 1

### Umsetzung von fremdsalzhaltigem, kristallinem Natriumdithionit mit Formalinlösung zu NaHMS

In einem Rührkessel werden 880g kristallines, 88 % Natriumdithionit (Hydrosulfit, BASF AG, Rest Fremdsalze), d.h. 4,5 mol Natriumdithionit in ca. 3,6 1 Wasser gelöst. Bei Raumtemperatur werden unter Rührung über 20 min 4,5 mol Formaldehyd (Formalin-Lösung, 30 %(w/w)) zugegeben. Nach Zugabe von 4,3 mol NaOH ist das Natriumdithionit quantitativ umgesetzt (online UV-Spektroskopie), der pH-Wert der Lösung liegt bei 9,7. Es werden insgesamt 4,31 mol NaHMS gebildet. Dies entspricht einer NaHMS-Ausbeute, bezogen auf das vorgelegte Dithionit, von 95 %.

### Beispiel 2

### Umsetzung von fremdsalzhaltiger Natriumdithionit-Lösung (Elektrolyseaustrag) mit Formalinlösung zu HMS

In einem Rührkessel wird ein wässriger Elektrolyseaustrag, der 2,24 mol Natriumdithionit und 0,6 mol Fremdsalze (NaHSO₃, Na₂SO₃) enthält, vorgelegt. Durch Zugabe von 50 % NaOH wird der pH-Wert auf pH > 8 und pH < 10 eingestellt. Bei Raumtemperatur werden anschliessend unter Rührung über 20 min 2,3 mol Formaldehyd (Formalin-Lösung, 30 %(w/w)) zugegeben. Durch Zugabe von 50 % (w/w) NaOH wird der End-pH-Wert auf pH = 11,5 eingestellt, das Natriumdithionit ist nach der Zugabe quantitativ umgesetzt (online UV-Spektroskopie). Es werden insgesamt 2,21 mol NaHMS gebildet. Dies entspricht einer NaHMS-Ausbeute, bezogen auf das vorgelegte Dithionit, von 98 %.

### Beispiel 3

### Elektrodialyse von einer Fremdsalz enthaltenden, wässrigen NaHMS-Lösung

In einer Elektrodialysezelle (Osmota 100, 5 Zellpaare, Elektroden aus RA2 und Titan-Streckmetall, Elektrolyt 5 % (w/w) Na₂CO₃, Kationentauscher CMX, Anionentauscher ASV, Deckmembran CMB) wird die aufzureinigende NaHMS und fremdsalzhaltige Reaktionslösung (89 g/l NaHMS, 116 g/l Na₂SO₃) im Diluatkreis vorgelegt. Der Produktkreis enthält bei Versuchbeginn eine stark verdünnte Salzlösung (5,6 g/l NaHMS, 10,6 g/l Na₂SO₃). Unter Umpumpung beider Kreise wird bei T = 35 °C für 17 h ein Strom von I = 1,81 A angelegt. Nach Versuch liegen im Diluatkreis folgende Konzentrationen vor: 5,4 g/l NaHMS und 120 g/l Na₂SO₃. Im Produktkreis betragen die Konzentrationen 94 g/l NaHMS und 27,3 g/l Na₂SO₃.

### Beispiel 4

### Methanolfällung einer Fremdsalz enthaltenden, wässrigen NaHMS-Lösung

Eine wässrige Lösung mit Gesamtmasse m = 1401,5 g, die 0,9 mol NaHMS und 1,35 mol Na₂SO₃ enthält, wird in einem gerührten Gefäß bei T = 40 °C mit 1401,5 g MeOH versetzt. Nach 30 min. werden die entstandenen Kristalle abgesaugt. Die verbleibende Mutterlauge enthält 0,876 mol NaHMS und 0,05 mol Na₂SO₃.

## Patentansprüche

1. Verfahren zur Herstellung von fremdsalzarmen Sulfinaten durch Reduktion von Dithionit-Salzen der Alkali- oder Erdalkalimetalle oder Ammonium mit Carbonylverbindungen oder Iminen bei einem pH-Wert > 6, vorzugsweise 8 bis 13, insbesondere 9 bis 11.

2. Verfahren nach Anspruch 1, **dadurch gekennzeichnet, dass** der pH-Wert durch Zugabe von Alkali- oder Erdalkalimetallhydroxiden oder Stickstoffbasen eingestellt wird, vorzugsweise durch Verwendung von NaOH.

3. Verfahren nach Anspruch 1 oder 2, **dadurch gekennzeichnet, dass** dies mit einem molaren Verhältnis Dithionitsalz zu Carbonyl- oder Iminverbindung von 0,75 bis 1,3 durchgeführt wird.

4. Verfahren nach Anspruch 3, **dadurch gekennzeichnet, dass** das molare Verhältnis Dithionit zu Carbonylverbindung oder Iminverbindung bei Werten von 0,95 bis 1,05, insbesondere 0,99 bis 1,02 liegt.

5. Verfahren nach einem der Ansprüche 1 bis 4, **dadurch gekennzeichnet, dass** Dithionit in Form des Na-Salzes eingesetzt wird.

6. Verfahren nach einem der Ansprüche 1 bis 5, **dadurch gekennzeichnet, dass** die Carbonylverbindung ausgewählt ist aus aliphatischen und aromatischen, ein- und mehrwertigen Aldehyden und Ketonen.

7. Verfahren nach einem der Ansprüche 1 bis 6, **dadurch gekennzeichnet, dass** als Iminverbindung von NH₃ abgeleitete Imine eingesetzt werden.

8. Verfahren nach einem der Ansprüche 1 bis 7, **dadurch gekennzeichnet, dass** als Carbonylverbindung Formaldehyd eingesetzt wird.

9. Verfahren nach einem der Ansprüche 1 bis 8, **dadurch gekennzeichnet, dass** Hydroxymethylsulfinat hergestellt wird.

10. Verfahren nach einem der Ansprüche 1 bis 9, **dadurch gekennzeichnet, dass** das eingesetzte Dithionit durch elektrolytische Reduktion von SO₂ oder Sulfiten hergestellt wurde.

11. Verfahren nach einem der Ansprüche 1 bis 10, **dadurch gekennzeichnet, dass** das erhaltene Sulfinat-Produkt durch Abtrennung entstandener Fremdsalze weiter aufgereinigt wird.

12. Verfahren nach Anspruch 11, **dadurch gekennzeichnet, dass** die Abtrennung durch Ausfällung der Fremdsalze mit polaren, wasserlöslichen organischen Lösungsmitteln und anschließender Trennung der flüssigen von der festen Phase geschieht.

13. Verfahren nach Anspruch 12, **dadurch gekennzeichnet, dass** die Lösungsmittel ausgewählt sind aus Ketonen, Alkoholen, Aldehyden, Amiden, Sulfoxiden, wasserlöslichen cyclischen Ethern und wasserlöslichen N-Heterocyclen

14. Verfahren nach Anspruch 13, **dadurch gekennzeichnet, dass** als Lösungsmittel ein Alkohol eingesetzt wird, insbesondere das Lösungsmittel Methanol, Ethanol oder Isopropanol ist.

15. Verfahren nach Anspruch 11, **dadurch gekennzeichnet, dass** die Abtrennung im elektrischen Gradienten, vorzugsweise Elektrodialyse oder Elektophorese, oder Abtrennung durch Druckgradienten, vorzugsweise Osmose, Nanofiltration, Umkehrosmose oder selektive Ionentauscher, erfolgt.
